# FASCICULE DE BREVET EUROPEEN

(11) **EP 1 943 573 B1**
(45) Date de publication et mention de la délivrance du brevet: **20.05.2009**
(21) Numéro de dépôt: 06794533.7
(22) Date de dépôt: 08.08.2006
(51) Int. Cl.: G05D 21/02, A23C 9/12

(54) **PROCEDE DE FABRICATION D'UN PRODUIT ALIMENTAIRE OU BIOTECHNOLOGIQUE METTANT EN OEUVRE UNE REGULATION DU POTENTIEL REDOX**
VERFAHREN ZUM HERSTELLEN EINES NAHRUNGSMITTEL- ODER BIOTECHNOLOGISCHEN PRODUKTS UNTER VERWENDUNG EINER REDOX-POTENTIALREGULIERUNG
METHOD FOR MAKING A FOOD OR BIOTECHNOLOGICAL PRODUCT USING REDOX POTENTIAL REGULATION

(30) Priorité: 30.09.2005 FR 0552977
(43) Date de publication de la demande: 16.07.2008
(73) Titulaire: L'AIR LIQUIDE, Société Anonyme pour l'Etude et l'Exploitation des Procédés Georges Claude, 75007 Paris (FR)
(72) Inventeur: GIRAULT, Christel, F-78960 Voisins le Bretonneux (FR); IBARRA, Dominique, F-91190 Gif sur Yvette (FR)
(74) Mandataire: Mellul-Bendelac, Sylvie Lisette
(86) Numéro de dépôt international: PCT/FR2006/050792
(87) Numéro de publication internationale: WO 2007/036653

(56) Documents cités:
- WO-A-98/27824
- FR-A- 2 848 122
- US-A- 5 453 286

## Description

La présente invention concerne le domaine des procédés de fabrication de produits alimentaires et biotechnologiques, mettant en oeuvre une ou des étapes de fermentation.

Les procédés envisagés sont très variés et peuvent être divisés en deux grandes familles :
- les procédés de fabrication de produits fermentés alimentaires tels que les produits laitiers fermentés (yaourts, fromages blancs etc...), les boissons fermentées (bières, vin etc...),
- les procédés de fabrication de biomasse (ferments lactiques, levures etc...) et de métabolites (ingrédients alimentaires, molécules d'intérêt telles que les enzymes, acides aminés, médicaments, éthanol...etc...).

L'invention s'attache à proposer de nouvelles conditions opératoires permettant selon le cas considéré d'améliorer notamment les propriétés des produits ainsi fabriqués, et notamment leurs propriétés microbiologiques, sensorielles, physico-chimiques etc..., ou bien les rendements, les puretés etc...

Comme on le verra ci-dessous plus en détails, la présente invention propose une nouvelle méthode de conduite de tels procédés par le contrôle et la régulation du potentiel redox du milieu considéré à un niveau donné de potentiel redox (niveau du redox défini dans chaque cas de façon à avoir un effet souhaité optimum), en un ou plusieurs points clés du procédé, ceci par des ajouts contrôlés d'un ou plusieurs gaz ou mélanges gazeux adaptés.

On peut obtenir des mesures du potentiel redox dans un milieu à l'aide de tout moyen disponible tel que par exemple les sondes redox qui permettent de faire des mesures directes et en continu dans des milieux liquides ou semi-liquides, ou encore par voie indirecte par des mesures de teneur en gaz dissous, par exemple d'hydrogène dissous dans le milieu.

Le suivi et la régulation du potentiel redox à certaines étapes clés du procédé permettent par exemple de réaliser des étapes ou phases du procédé en conditions réductrices stables ou d'alterner durant le procédé des étapes en conditions réductrices avec des étapes en conditions oxydantes.

Une condition est considérée oxydante ou réductrice par rapport au potentiel redox du milieu avant l'ajustement par les gaz. Ainsi une condition sera dite réductrice quand le potentiel d'oxydoréduction sera inférieur à la valeur initiale avant son ajustement et sa régulation (que le potentiel atteint soit négatif ou non). Inversement, une condition sera dite oxydante quand le potentiel d'oxydoréduction sera supérieur à la valeur initiale avant son ajustement et sa régulation (que le potentiel atteint soit positif ou non).

On rappellera que les oxydo-réductions sont des étapes essentielles dans les réactions de l'anabolisme et du catabolisme cellulaire, pour lesquelles le sens des échanges est déterminé par le potentiel d'oxydo-réduction (ci-après Eh). Le Eh est un paramètre d'état des fermentations ; sa variation modifie l'environnement physico-chimique des microorganismes. Les activités métaboliques et la physiologie des microorganismes sont déterminées par le pH intracellulaire (PHᵢₙ) qui va conditionner l'activité des enzymes et l'accessibilité de certains substrats et cofacteurs dans les réactions du métabolisme. Le pHᵢₙ est fonction du pH extracellulaire (pHₑₓ) et de l'aptitude du microorganisme à maintenir une certaine homéostasie cellulaire. La différence entre le pHᵢₙ et le pHₑₓ va également modifier la valeur de la force proto-motrice ΔµH⁺, qui est notamment impliquée dans les échanges de la cellule microbienne avec l'extérieur. Les paramètres Eh et pHᵢₙ sont intimement liés ; ainsi l'énergie retrouvée dans les composés à haut potentiel comme d'adénosine tri-phosphate (ATP) et gagée par le catabolisme des substrats pourra être utilisée par la cellule pour maintenir son pHᵢₙ (et donc son ΔpH) grâce aux ATPases membranaires.

Selon Urbach et al en 1995 (« Contribution of lactic acid bacteria to flavour compound formation in dairy products", International Dairy Journal. 5: 877-903), les bactéries lactiques sont largement impliquées dans la production des arômes des produits laitiers fermentés ; elles convertissent le lactose en acide lactique ; ceci conduit à la production de diacétyle et d'acétaldéhyde qui sont les principaux arômes des laits fermentés et des fromages frais. Le Eh est un paramètre environnemental qui va pouvoir conditionner les activités métaboliques des microorganismes et notamment leur capacité à synthétiser des molécules d'arômes. En particulier, il a été montré pour l'emmental et le cheddar, que les fromages de bonne qualité avaient un faible potentiel d'oxydoréduction.

Le Eh est un paramètre physico-chimique qui, de par sa nature, agit sur tous les milieux, pourvu que ceux-ci contiennent au moins une molécule qui puisse passer d'un état oxydé à réduit et vice versa. C'est pourquoi son effet est perceptible sur toutes les fonctions cellulaires. Son action a été montrée sur différents types de souches bactériennes, à titre illustratif :
- L'ajout de réducteurs chimiques dans les milieux de culture a permis de modifier significativement la croissance et les flux métaboliques chez *Corynebacterium glutamicum*, *Clostridium acetobutylicum, Sporidiobolus* ruinenii et *Escherichia coli* ;
- Un Eh réducteur fixé par des gaz a permis de modifier les flux métaboliques chez *Saccharomyces cerevisiae* avec une augmentation du ratio glycérol/éthanol et l'accumulation des sucres de réserve avec augmentation de la survie des levures lors de la conservation.

En milieu industriel, le Eh est déjà indirectement pris en compte au travers de l'oxygène dont l'effet inhibiteur sur les bactéries lactiques est bien identifié. Cet effet est dû à leur incapacité à synthétiser des cytochromes et les: enzymes à noyau hème.

On sait par ailleurs qu'il est aussi possible en agissant sur le Eh de modifier la survie des ferments probiotiques, les flux métaboliques, la production et/ou la stabilité des molécules d'arômes. L'ensemble de ces résultats a été obtenu suite à une modification du Eh par les microorganismes eux-mêmes, par des molécules oxydo-réductrices, ou par traitement thermique.

Dans le domaine de l'utilisation de mélanges gazeux dans les milieux de fermentation de bactéries lactiques on peut citer également les travaux de Henriksen et al parus dans Letters in Applied Microbiology en 2000 (vol. 30 p415-418) qui se sont intéressés à la croissance de bactéries lactiques, et ont montré que lorsque les cultures étaient balayées par de l'azote la croissance était fortement ralentie, alors que l'ajout, d'infimes quantités de CO₂ dans ce cas faisait redémarrer la croissance sous forme exponentielle.

La demande de brevet français FR-A-2 848 122 donne un exemple de procédé de fabrication d'un produit alimentaire, mettant en oeuvre un milieu, au cours duquel on procède, à une opération de contrôle du potentiel redox du milieu, le procédé comprenant une étape dans laquelle on régule à un niveau prédéterminé de consigne le potentiel redox du milieu.

La présente invention concerne alors un procédé de fabrication d'un produit alimentaire ou biotechnologique, mettant en oeuvre une ou plusieurs étapes, l'une ou plusieurs des étapes mettant en oeuvre un milieu, l'une ou plusieurs des étapes mettant en oeuvre un milieu étant une étape de fermentation, au cours duquel on procède, durant au moins l'une des étapes du procédé à une opération de contrôle du potentiel redox du milieu de l'étape considérée, et se **caractérisant en ce que** l'on effectue la conduite du procédé de la manière suivante :
- on régule à un niveau prédéterminé de consigne le potentiel redox du milieu de l'étape dont le potentiel redox est contrôlé à l'aide d'ajouts contrôlés d'un gaz de traitement dans le milieu considéré, et
- on autorise le passage à l'étape suivante de ladite étape considérée dans le procédé lorsque ladite valeur de consigne est atteinte,
   de façon à réaliser au moins une des dites étapes du procédé en condition réductrice et au moins une des dites étapes du procédé en condition oxydante.

On parlera indifféremment dans ce qui suit d'étapes ou de phases constituant le procédé, ou encore de phases constituant une étape du procédé.

Le procédé selon l'invention pourra par ailleurs adopter l'une ou plusieurs des caractéristiques techniques suivantes :
- le procédé est un procédé de fabrication d'un produit laitier fermenté, et la régulation du potentiel redox a lieu en plusieurs des étapes de façon à séquencer les phases d'oxydation et de réduction de la façon suivante :
   - on effectue une régulation du potentiel redox de façon à mettre en place des conditions réductrices en un ou plusieurs points du procédé situés en amont de la phase de pasteurisation ;
   - on effectue une régulation du potentiel redox de façon à mettre en place des conditions oxydantes en un ou plusieurs points du procédé situés en aval de la pasteurisation.
- le procédé est un procédé de fabrication d'un produit laitier fermenté, et la régulation du potentiel redox a lieu en plusieurs des étapes de façon à séquencer les phases d'oxydation et de réduction de la façon suivante :
   - on effectue une régulation du potentiel redox de façon à mettre en place des conditions oxydantes en un ou plusieurs points du procédé situés en amont de la phase de pasteurisation ;
   - on effectue une régulation du potentiel redox de façon à mettre en place des conditions réductrices en un ou plusieurs points du procédé situés en aval de la pasteurisation.
- le produit laitier fermenté est un yaourt.
- ladite conduite du procédé est réalisée de manière à ce que, pour au moins l'une des dites étapes de fermentation, des ajouts contrôlés d'un gaz de traitement permettent d'alterner des phases de la fermentation considérée en conditions réductrices avec des phases de la fermentation considérée en conditions oxydantes.
- le procédé est un procédé de fabrication de bière, et ladite conduite du procédé permet que la régulation du potentiel redox pendant la fermentation ait lieu en deux temps : dans un premier temps, la fermentation a lieu en condition oxydante régulée et en présence d'oxygène, pour favoriser la croissance de la levure et son bon état physiologique, et dans un second temps, le potentiel redox est abaissé à une valeur optimale pour permettre d'améliorer les paramètres de fermentation, ainsi que les critères sensoriels.
- le procédé est un procédé de fermentation en fermenteur visant à la fabrication de biomasse et/ou de métabolites, et ladite conduite du procédé permet de réguler le potentiel redox du milieu à différentes valeurs successives en fonction des différentes phases de la fermentation de façon à réaliser une première phase en conditions oxydantes afin de favoriser la croissance de la souche microbienne par des conditions oxydantes, et après l'obtention d'une teneur maximale en biomasse de basculer le fermenteur en conditions réductrices afin d'initier ou d'intensifier la production d'un ou plusieurs métabolites recherchés.
- le procédé est un procédé de fermentation en fermenteur visant à la fabrication de biomasse et/ou de métabolites, et ladite conduite du procédé permet de réguler le potentiel redox du milieu à différentes valeurs successives en fonction des différentes phases de la fermentation, de façon à réaliser une première phase rendue légèrement réductrice qui est favorable à la croissance de certains microorganismes et donc à la production d'une biomasse importante, suivie par une phase plus réductrice qui va permettre de favoriser la production de composés d'arômes souhaités.
- le procédé est un procédé de fermentation en fermenteur visant à la fabrication de biomasse et/ou de métabolites, et ladite conduite du procédé permet de modifier et réguler le potentiel redox du milieu à une valeur différente en fin de fermentation afin d'adapter le métabolisme ou la physiologie des micro-organismes pour les préparer à une étape ultérieure.
- le procédé est un procédé de fermentation en fermenteur visant à la fabrication de biomasse et/ou de métabolites, et ladite conduite du procédé permet de changer le niveau de potentiel redox après la production d'un métabolite désiré pour favoriser l'excrétion du métabolite considéré dans le milieu de récupération.
- le procédé est un procédé de fermentation en fermenteur visant à la fabrication de biomasse et/ou de métabolites, et ladite conduite du procédé permet de changer le niveau de potentiel redox après la production d'un précurseur d'une molécule d'intérêt pour favoriser une réaction chimique permettant l'obtention de la molécule d'intérêt recherchée.
- le procédé est un procédé de fermentation en fermenteur visant à la fabrication de biomasse et/ou de métabolites, et en ce que postérieurement à la fermentation sont réalisées :

- des étapes de centrifugation, et/ou filtration et/ou ultrafiltration visant à récupérer la biomasse produite, ou
- des étapes de purification sur le milieu de fermentation pour séparer et concentrer des métabolites,
   et ladite conduite du procédé permet de changer le niveau de potentiel redox après la séparation du milieu contenant la molécule d'intérêt de la biomasse (cellules microbiennes) pour favoriser la séparation sélective de la molécule d'intérêt des autres composés du milieu en privilégiant par exemple la fixation sur une résine soit sous la forme réduite soit sous la forme oxydée de la molécule d'intérêt et son élution par une solution adaptée.

Il est donc proposé de contrôler le potentiel redox à des endroits clés dans le procédé, à l'aide de tout moyen disponible tel que par exemple les sondes redox. Ce contrôle permet d'ajuster le potentiel redox et de maîtriser l'ajout de gaz dans le milieu, tout en déterminant avec précision le moment où il est possible de stopper le processus de modification du redox.

Il est possible d'envisager un système de régulation automatique du potentiel redox du milieu, puisque l'on sait par exemple que certains microorganismes, de par leur activité, modifient le potentiel redox d'un milieu. Il est ainsi permis de garder un redox stable pendant une période donnée d'une fermentation.

Il peut être également possible, selon le procédé considéré, d'alterner des phases de conditions réductrices et oxydantes de façon à privilégier à des moments définis dans le procédé la mise en place de réactions biochimiques et/ou biologiques qui par leur enchaînement permet l'obtention d'un produit à caractéristiques définies. Ainsi à titre illustratif, il peut être intéressant de conduire une fermentation avec une première phase rendue légèrement réductrice par ajout d'azote (chassant une partie de l'oxygène dissous) qui est favorable à la croissance de certains microorganismes et donc à la production d'une biomasse importante, suivie par une phase plus réductrice (par exemple à l'aide d'un mélange contenant de l'hydrogène) qui va permettre par exemple de favoriser la production de composés d'arômes souhaités tels que par exemple l'acétaldéhyde produit par les bactéries lactiques.

Comme on l'aura compris à la lecture de ce qui précède, le gaz de traitement devra être choisi en fonction du procédé considéré, de l'étape considérée, des conditions redox que l'on souhaite atteindre, et l'on pourra donc envisager d'utiliser un gaz neutre tel que l'azote, l'argon, l'hélium, ou le dioxyde de carbone, mais aussi un gaz oxydant tel que l'oxygène ou l'air, ou encore un gaz réducteur tel que l'hydrogène, ou encore un mélange de tels gaz.

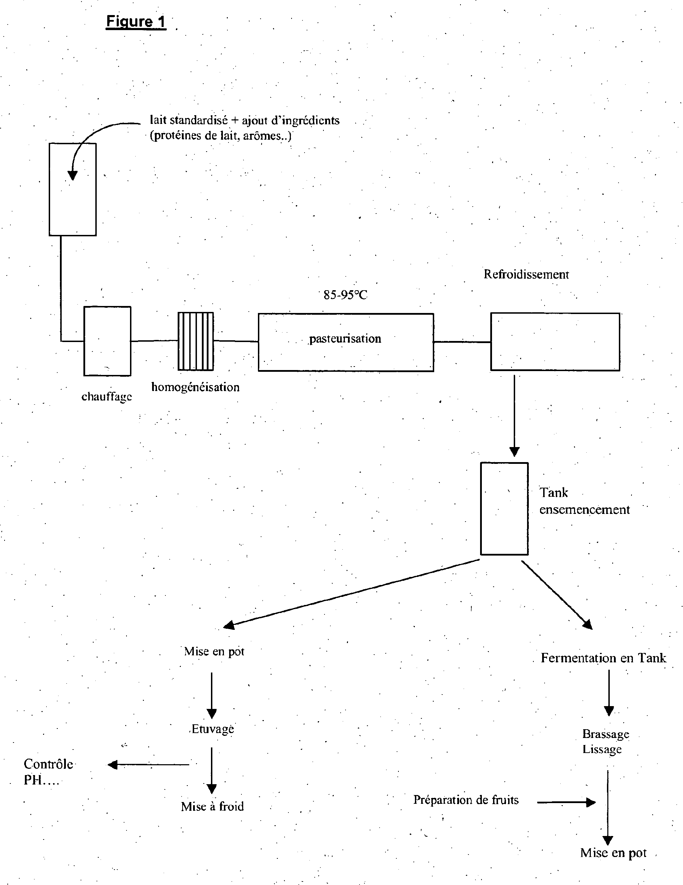

D'autres caractéristiques et avantages ressortiront de la description suivante, donnée uniquement à titre d'exemple et faite notamment en référence à la figure 1 annexée qui illustre un exemple d'application de l'invention au cas d'une installation de fabrication de yaourts ou autres produits laitiers fermentés.

On reconnaît sur la figure 1 les éléments et étapes suivantes :
- une préparation du lait qui comprend en général les additions de composants tels que matières grasses ou protéines ou encore arômes pour en fixer la composition ;
- une étape de chauffage ;
- une étape de pasteurisation (traitement thermique) ;
- une homogénéisation (qui peut avoir lieu avant ou après la pasteurisation) ;
- un refroidissement jusqu'à la température à laquelle aura lieu l'ensemencement;
- l'ensemencement avec les souches souhaitées ;
- selon le type de yaourt ou produit laitier fermenté considéré : une fermentation en tank suivie d'un lissage avant une mise en pot (ex : yaourts dits « brassés »), ou bien la répartition préalable du mélange ensemencé en pots où s'effectuera la fermentation (ex : yaourts dits « fermes » ou « étuvés »)
- un refroidissement des produits avant leur stockage.

On peut envisager selon d'effectuer une régulation du potentiel redox aux points suivants de la chaîne :
i) dans le lait de départ avant standardisation,
j) dans le mélange laitier résultant de l'ajout des ingrédients additionnels dans le lait ;
k) avant ou après l'étape d'homogénéisation dudit mélange laitier ;
l) avant ou après l'étape de pasteurisation du mélange laitier ;
m) avant ou après l'étape d'ensemencement du mélange laitier avec une ou plusieurs souches de bactéries lactiques ;
n) avant le conditionnement du produit en emballage final.

Conformément à l'invention, dans ce cas de la fabrication de yaourts ou produits laitiers fermentés on peut envisager de séquencer les phases d'oxydation et de réduction. En effet, sans être à aucun moment lié par l'explication qui suit, on peut penser que les valeurs réductrices pourront favorablement jouer sur la conformation des protéines, notamment les protéines sériques, riches en molécules soufrées. Le potentiel redox permet de jouer sur l'état de ces molécules qui se trouvent soit sous forme de groupements thiol, soit sous forme de ponts disulfures, et jouent un rôle crucial dans la formation du réseau protéique après dénaturation des protéines lors de l'étape de pasteurisation. L'utilisation de conditions réductrices en amont de la phase de pasteurisation permettra donc de coupler cet effet à l'effet de la chaleur sur le protéines ce qui aura pour conséquence de réaliser des gels protéiques de structure stable qui seront favorables notamment à une limitation des phénomènes de synérèse dans les yaourts. En aval de la pasteurisation, il sera alors avantageux de rétablir des valeurs redox moins réductrices (ou égales aux valeurs habituelles du lait) qui permettront aux bactéries ensemencées de se développer normalement sans influencer leur métabolisme et donc sans conséquence organoleptique.

Il peut également être avantageux d'ajuster une valeur cible du potentiel redox lors de la fermentation pour influencer de manière volontaire le métabolisme des ferments lactiques, et ainsi par exemple orienter la production d'arômes vers les composés souhaités.

En basculant pour finir le potentiel redox du produit fini à une valeur plus réductrice que celle de la fermentation, on pourra stabiliser microbiologiquement le yaourt et ainsi mieux le préserver du développement éventuel de certaines levures ou moisissures.

On a plus particulièrement développé dans ce qui précède les exemples de la fabrication de yaourts mais l'on peut également évoquer le cas de la fabrication de boissons fermentées telles que la bière ainsi que le cas de productions de produits en fermenteurs. C'est ce qui sera fait ci-dessous.

### Evoquons donc à présent le cas de la fabrication de la bière.

On va montrer ci-dessous le fait que dans le cas de la fabrication de la bière, il est intéressant de faire en sorte que la régulation du potentiel redox pendant la fermentation ait lieu en deux temps, en deux phases. Dans un premier temps, la fermentation a avantageusement lieu en condition oxydante régulée et en présence d'oxygène, pour favoriser la croissance de la levure et son bon état physiologique. Dans un second temps, le potentiel redox est abaissé à une valeur optimal pour permettre d'améliorer les paramètres de fermentation, ainsi que les critères sensoriels (arômes, tenue de la mousse).

Rappelons que le procédé brassicole comporte typiquement deux étapes de fermentation :
- La fermentation principale : après aération du moût, celui-ci est ensemencé avec une levure du genre *Saccharomyces*, qui va, par fermentation, transformer les sucres fermentescibles en alcool et en gaz carbonique.
- La fermentation secondaire ou « garde » : cette étape se déroule à la suite de la précédente en abaissant la température du milieu à une température proche de 0°C pendant une durée qui varie de quelques jours à quelques semaines. La bière jeune va se saturer en dioxyde de carbone, ce, qui contribuera très fortement à son caractère moussant. C'est aussi pendant cette phase de maturation que la bière se clarifie et que sa flaveur s'affine.

Les exemples qui suivent vont montrer qu'il est avantageux de fermenter le moût par moments choisis en condition oxydante et par moments choisis en condition réductrice.

Exemple 1 : Six tests de fermentation de moût, de type « Kirin », ont été réalisés en utilisant un extrait de malt (ce test est bien connu de l'homme du métier, c'est un test prédictif, autonome, i.e déconnecté du procédé global de fabrication de la bière) : 2 moûts témoins (potentiel redox moyen : 400mV), 2 moûts dont le potentiel redox a été réduit par un bullage initial à l'azote (potentiel redox moyen : 140mV), et 2 moûts dont le potentiel redox a été réduit par un bullage initial avec un mélange azote/hydrogène (96/4) (potentiel redox moyen : -415mV). Les moûts ont été ensemencés par une levure sèche active à 11.10⁶ cellules viables par mL. L'objectif était de déterminer l'influence du potentiel redox sur la performance fermentaire de la levure, pendant 8 jours à 8°C.

Les paramètres suivants ont été suivis :
- la décroissance de l'extrait fermentescible pendant 8 jours,
- l'évolution de la turbidité du moût en fermentation (croissance de la levure) par la mesure de la densité optique du moût à 800nm pendant 8 jours,
- les paramètres de fermentation : extrait apparent (extrait fermentescible plus extrait non fermentescible) du moût en fin de test, atténuation apparente (pourcentage d'extrait fermentescible utilisé parla levure par rapport à l'extrait total du moût), degré d'atténuation (proportion de l'extrait fermentescible utilisé par la levure par rapport à l'extrait fermentescible total).

Les résultats (rapportés au niveau du tableau 1) ont montré que la baisse du potentiel redox du moût entraîne une amélioration des paramètres de fermentation: diminution de l'extrait apparent, augmentation du degré d'atténuation et de l'atténuation apparente, et augmentation de la densité optique. Ces résultats traduisent une utilisation plus importante de sucres fermentescibles et une croissance plus importante de la levure en conditions plus réductrices.

**Tableau 1 : Valeurs moyennes des résultats des tests de fermentation**

| | Témoin | Condition N₂ | Condition N₂/H₂ |
|---|---|---|---|
| Extrait apparent au 8^{ème} jour (degré Plato) | 3.27 | 3.04 | 2.62 |
| Atténuation apparente au 8^{ème} jour (%) | 70.3 | 72.4 | 76.0 |
| Degré d'atténuation (%) | 90.8 | 93.4 | 97.8 |
| Densité optique au 8^{ème} jour (à 800nm) | 1.22 | 1.5 | 1.52 |

Exemple 2 : Six tests de micro-brassage (micro-fabrication de bière) dé 30 litres ont été réalisés de la façon suivante : 2 moûts témoins aérés (potentiel redox moyen : 291mV), 2 moûts dont le potentiel redox a été réduit par un bullage initial à l'azote (potentiel redox moyen : 216mV), et 2 moûts dont le potentiel redox a été réduit par un bullage initial avec un mélange azote/hydrogène (96/4) (potentiel redox moyen : -290mV). Les bières ainsi obtenues ont été analysées. En résumé, le potentiel redox du moût avant chauffage est contrôlé, le reste de la chaîne de fabrication est traditionnel.

Les résultats présentés dans le tableau 2 montrent que modifier le potentiel redox permet d'améliorer la tenue de la mousse. En effet plus le potentiel redox est faible plus la mousse tient longtemps.

Par ailleurs, l'analyse sensorielle (réalisée sous forme de tests triangulaires) montre des différences significatives (au seuil de 5%) entre la bière témoin et celle obtenue après réduction du potentiel redox par le mélange N₂/H₂. Les descripteurs cités (oxydé, forte acidité) sur la bière témoin dénotent un certain état d'oxydation de la bière, plus prononcé que sur la bière obtenue avec le mélange N₂/H₂.

En revanche, on note une légère tendance à l'augmentation de la teneur en SO₂ des bières issues des moûts avec réduction du potentiel redox par N₂ ou N₂/H₂. La teneur en SO₂ augmente en général en fonction du mauvais état physiologique des levures. L'augmentation observée ici s'explique certainement par le manque d'aération dé la levure.

Les conditions réductrices ont donc amélioré la qualité sensorielle de la bière, sans toutefois améliorer les paramètres de fermentation de la façon décrite en exemple 1.

**Tableau 2 : Résultats d'analyse des bières (valeurs moyennes des 2 répétitions pour la tenue de mousse, et valeurs des 2 répétitions pour la teneur en SO₂).**

| | Témoin | | Condition N₂ | | Condition N₂/H₂ | |
|---|---|---|---|---|---|---|
| Tenue de mousse (s) (méthode NIBEM) | 266 | | 278 | | 293 | |
| SO₂ (mg/l) | < 1 | < 1 | < 1 | 3.8 | < 1 | 2.6 |

On a donc bien montré par ce qui précède que dans le cas de la fabrication de la bière, il est intéressant de faire en sorte que la régulation du potentiel redox pendant la fermentation ait lieu en deux temps. Dans un premier temps, la fermentation a lieu en condition oxydante régulée et en présence d'oxygène, pour favoriser la croissance de la levure et son bon état physiologique. Dans un second temps, lé potentiel redox est abaissé à une valeur optimale pour permettre d'améliorer les paramètres de fermentation, ainsi que les critères sensoriels (arômes, tenue de la mousse).

Evoquons maintenant ci-dessous le cas des productions en fermenteurs.

D'une façon générale, les productions en fermenteurs répondent à deux besoins, de façon distincte ou simultanée la production de biomasse et/ou la production de molécules d'intérêt. Plusieurs objectifs peuvent être recherchés :
- la production de biomasse,
- la production de biomasse couplée à celle de molécules d'intérêt (métabolites primaires),
- la production de molécules d'intérêt autres que celles produites en même temps que la biomasse, c'est-à-dire les métabolites secondaires,
- l'enchaînement de la production de biomasse puis de molécules d'intérêt.

Il existe différents types de culture :
- la culture discontinue ou batch pour laquelle, le milieu de culture est apporté en une seule fois au début, sans qu'il y ait apport ou extraction de milieu au cours de la fermentation,
- la culture semi-continue ou « fed-batch », utilisée par exemple pour la production de biomasse sensible à l'inhibition par le substrat, et pour laquelle l'apport continu ou périodique de nutriments est couplé à la croissance, sans qu'aucun prélèvement continu de milieu ne soit réalisé,
- la culture continue, pour laquelle les microorganismes sont maintenus en phase exponentielle de croissance par l'apport continu d'un milieu neuf qui équilibre le prélèvement continu de milieux contenant les cellules en suspension.

Ces fermentations, qu'elles soient aérobies ou anaérobies, ont lieu en fermenteur ou biréacteur, avec ou sans agitation, dans un milieu dont la composition est définie de façon à orienter la fermentation vers la production voulue. De même, les paramètres de fermentation tels que le pH, la température, la pression d'oxygène dissous, la vitesse d'agitation etc... sont en général régulés à des valeurs optimum pré-déterminées de façon à maximiser la production recherchée.

On sait que le potentiel redox peut-être en perpétuelle évolution au cours d'une fermentation pour une ou plusieurs des raisons suivantes :
- la croissance des microorganismes,
- la production de molécules oxydantes ou réductrices par les microorganismes,
- l'apport de milieu de culture neuf dans le cas des cultures semi-continues et continues.

La présente invention propose alors de contrôler en permanence le potentiel redox et de le réguler à sa valeur optimum par l'utilisation d'ajouts contrôlés de gaz. Cette valeur optimum est pré-déterminée expérimentalement de façon à optimiser la réaction recherchée à un moment donné de la fermentation.

Avantageusement, on pourra associer ou non à cette régulation un ajustement du potentiel redox du milieu de culture neuf apporté dans le cas des cultures semi-continues et continues.

Selon un des aspects de l'invention, on régule le potentiel redox du milieu à différentes valeurs successives en fonction des différentes périodes de la fermentation, de façon à piloter celle-ci pour l'orienter vers telle ou telle réaction souhaitée.

La fermentation peut ainsi être régulée en condition réductrice dans un premier temps puis être régulée en condition plus oxydante dans un second temps et inversement :
- on peut par exemple choisir de favoriser la croissance de la souche microbienne par des conditions oxydantes et après l'obtention d'une teneur maximale en biomasse basculer le fermenteur en conditions plus réductrices afin d'initier ou d'intensifier la production d'un ou plusieurs métabolites recherchés. On sait par exemple que *Saccharomyces se* développe mieux en conditions oxydantes et produit plus de glycérol en conditions réductrices. De même on sait que des cultures de *Corynebacterium glutamicum* produisent plus d'acides aminés en conditions réductrices ou que *Sporidiobolus* produit plus de composés d'arômes en milieu réducteur.
- il est également possible, en régulant le potentiel redox du milieu à une valeur différente en fin de fermentation, d'adapter le métabolisme ou la physiologie des micro-organismes pour les préparer à une étape ultérieure. II peut ainsi être intéressant par exemple de produire *Saccharomyces cerevisiae* en présence d'oxygène et de basculer le milieu de production de cette levure à un niveau réducteur optimum afin de-l'adapter à son utilisation future, comme par exemple la panification.

On peut ainsi piloter la fermentation à 2 ou 3 valeurs de redox successives, voir plus selon les besoins. Ainsi on peut envisager de rechanger le niveau de régulation après la production du métabolite désiré (par exemple la production d'une enzyme pariétale) avant sa récolte ou séparation du milieu de culture et donc en fin de fermentation, pour favoriser l'excrétion du métabolite considéré dans le milieu de récupération. Ce changement de potentiel redox peut également intervenir après la production d'un précurseur de la molécule d'intérêt qui en condition réductrice subira une réaction chimique permettant l'obtention de la molécule d'intérêt en question.

On sait par ailleurs qu'après la fermentation proprement dite, plusieurs procédés peuvent être appliqués. Des étapes de centrifugation, filtration ou ultrafiltration permettent de récupérer la biomasse produite. Des étapes de purification peuvent être réalisées sur le milieu de fermentation pour séparer et concentrer des métabolites centrifugation, filtration, chromatographies, précipitation par ajout de sels ou solvant etc...

L'invention propose alors de réguler le redox pour favoriser la séparation de ces molécules d'intérêt. Par exemple la production et/ou l'excrétion dans le milieu de culture a lieu en condition oxydante (ou réductrice), et on bascule en condition réductrice (respectivement oxydante) pour conduire l'étape de séparation. Ainsi par exemple, une molécule peut être retenue dans une colonne de chromatographie en se fixant à une résine en condition oxydante et être ensuite relarguée dans une solution d'élution réductrice.

Si dans les procédés visés par la présente invention, comprenant une ou plusieurs étapes de fermentation, l'ensemencement du milieu peut être effectué de façon directe, on peut également l'envisager de manière indirecte par le fait que l'on effectue au préalable une ou plusieurs pré-cultures successives afin de constituer l'inoculum qui servira à ensemencer le milieu de fermentation; et on peut alors également envisager selon la présente invention le fait de procéder à un contrôle et une régulation du potentiel redox de la préculture à l'aide d'ajouts contrôlés d'un gaz de traitement dans le milieu de pré-culture considéré.

## Revendications

1. Procédé de fabrication d'un produit alimentaire ou biotechnologique, mettant en oeuvre une ou plusieurs étapes, l'une ou plusieurs des étapes mettant en oeuvre un milieu, l'une ou plusieurs des étapes mettant en oeuvre un milieu étant une étape de fermentation, au cours duquel on procède, durant au moins l'une des étapes du procédé à une opération de contrôle du potentiel redox du milieu de l'étape considérée, et **se caractérisant en ce que** l'on effectue la conduite du procédé de la manière suivante :
- on régule à un niveau prédéterminé de consigne le potentiel redox du milieu de l'étape dont le potentiel redox est contrôlé à l'aide d'ajouts contrôlés d'un gaz de traitement dans le milieu considéré, et
- on autorise le passage à l'étape suivante de ladite étape considérée dans le procédé lorsque ladite valeur de consigne est atteinte,
de façon à réaliser au moins une des dites étapes du procédé en condition réductrice et au moins une des dites étapes du procédé en condition oxydante.

2. Procédé selon la revendication 1, **caractérisé en ce que** le procédé est un procédé de fabrication d'un produit laitier fermenté, et **en ce que** la régulation du potentiel redox a lieu en plusieurs des étapes de façon à séquencer les phases d'oxydation et de réduction de la-façon suivante :
- on effectue une régulation du potentiel redox de façon à mettre en place des conditions réductrices en un ou plusieurs points du procédé situés en amont de la phase de pasteurisation ;
- on effectue une régulation du potentiel redox de façon à mettre en place des conditions oxydantes en un ou plusieurs points du procédé situés en aval de la pasteurisation.

3. Procédé selon la revendication 1, **caractérisé en ce que** le procédé est un procédé de fabrication d'un produit laitier fermenté, et **en ce que** la régulation du potentiel redox a lieu en plusieurs des étapes de façon à séquencer les phases d'oxydation et de réduction de la façon suivante :
- on effectue une régulation du potentiel redox de façon à mettre en place des conditions oxydantes en un ou plusieurs points du procédé situés en amont de la phase de pasteurisation ;
- on effectue une régulation du potentiel redox de façon à mettre en place des conditions réductrices en un ou plusieurs points du procédé situés en aval de la pasteurisation.

4. Procédé selon la revendication 2 ou 3 **caractérisé en ce que** le produit laitier fermenté est un yaourt.

5. Procédé selon la revendication 1, **caractérisé en ce que** ladite conduite du procédé est réalisée de manière à ce que, pour au moins l'une des dites étapes de fermentation, des ajouts contrôlés d'un gaz de traitement permettent d'alterner des phases de la fermentation considérée en conditions réductrices avec des phases de la fermentation considérée en conditions oxydantes.

6. Procédé selon la revendication 5, **caractérisé en ce que** le procédé est un procédé de fabrication de bière, et **en ce que** ladite conduite du procédé permet que la régulation du potentiel redox pendant la fermentation ait lieu en deux temps : dans un premier temps, la fermentation a lieu en condition oxydante régulée et en présence d'oxygène, pour favoriser la croissance de la levure et son bon état physiologique, et dans un second temps, le potentiel redox est abaissé à une valeur optimale pour permettre d'améliorer les paramètres de fermentation, ainsi que les critères sensoriels.

7. Procédé selon la revendication 5, **caractérisé en ce que** le procédé est un procédé de fermentation en fermenteur visant à la fabrication de biomasse et/ou due métabolites, et **en ce que** ladite conduite du procédé permet de réguler le potentiel redox du milieu à différentes valeurs successives en fonction des différentes phases de la fermentation de façon à réaliser une première phase en conditions oxydantes afin de favoriser la croissance de la souche microbienne par des conditions oxydantes, et après l'obtention d'une teneur maximale en biomasse de basculer le fermenteur, en conditions réductrices afin d'initier ou d'intensifier la production d'un ou plusieurs métabolites recherchés.

8. Procédé selon la revendication 5, **caractérisé en ce que** le procédé est un procédé de fermentation en fermenteur visant à la fabrication de biomasse et/ou de métabolites, et **en ce que** ladite conduite du procédé, permet de réguler le potentiel redox du milieu à différentes valeurs successives en fonction des différentes phases de la fermentation, de façon à réaliser une première phase rendue légèrement réductrice qui est favorable à la croissance de certains microorganismes et donc à la production d'une biomasse importante, suivie par une phase plus réductrice qui va permettre de favoriser la production de composés d'arômes souhaités.

9. Procédé selon la revendication 5, **caractérisé en ce que** le procédé est un procédé de fermentation en fermenteur visant à la fabrication de biomasse et/ou de métabolites, et **en ce que** ladite conduite du procédé permet de modifier est réguler le potentiel redox du milieu à une valeur différente en fin de fermentation afin d'adapter le métabolisme ou la physiologie des micro-organismes pour les préparer à une étape ultérieure.

10. Procédé selon la revendication 5, **caractérisé en ce que** le procédé est un procédé de fermentation en fermenteur visant à la fabrication de biomasse et/ou de métabolites, et **en ce que** ladite conduite du procédé permet de changer le niveau de potentiel redox après la production d'un métabolite désiré pour favoriser l'excrétion du métabolite considéré dans le milieu de récupération.

11. Procédé selon la revendication 5, **caractérisé en ce que** le procédé est un procédé de fermentation en fermenteur visant à la fabrication de biomasse et/ou de métabolites, et **en ce que** ladite conduite du procédé permet de changer le niveau de potentiel redox après la production d'un précurseur d'une molécule d'intérêt pour favoriser une réaction chimique permettant l'obtention de la molécule d'intérêt recherchée.

12. Procédé selon l'une des revendications 5 à 11, **caractérisé en ce que** le procédé est un procédé de fermentation en fermenteur visant à la fabrication de biomasse et/ou de métabolites, et **en ce que** postérieurement à la fermentation sont réalisées :
- des étapes de centrifugation, et/ou filtration et/ou ultrafiltration visant à récupérer la biomasse produite, ou
- des étapes de purification sur le milieu de fermentation pour séparer et concentrer des métabolites,
et **en ce que** ladite conduite du procédé permet de changer le niveau de potentiel redox après la séparation du milieu contenant la molécule d'intérêt de la biomasse pour favoriser la séparation sélective de la molécule d'intérêt des autres composés du milieu en privilégiant par exemple la fixation sur une résine soit sous la forme réduite soit sous la forme oxydée de la molécule d'intérêt et son élution par une solution adaptée.

## Claims

1. Process for producing a food or biotechnological product, implementing one or more steps, wherein one or more of the steps uses a medium, one or more of the steps that uses a medium being a fermentation step, said process consisting, during at least one of the steps thereof, in controlling the redox potential of the medium of the step in question, and being **characterized in that** it is conducted in the following way:
- regulating at a predetermined setpoint level the redox potential of the medium of the step of which the redox potential is controlled with controlled additions of a process gas to the medium in question, and
- proceeding to the step that follows said step in question in the process when said setpoint value is reached,
so as to carry out at least one of said steps of the process under reducing conditions and at least one of said steps of the process under oxidizing conditions.

2. Process according to Claim 1, **characterized in that** the process is a process for producing a fermented milk product, and **in that** the regulating of the redox potential is carried out in several of the steps so as to sequence the oxidation and reduction phases in the following way:
- the redox potential is regulated so as to set up reducing conditions at one or more points of the process located upstream of the pasteurization phase;
- the redox potential is regulated so as to set up oxidizing conditions at one or more points of the process located downstream of the pasteurization.

3. Process according to Claim 1, **characterized in that** the process is a process for producing a fermented milk product, and **in that** the regulating of the redox potential is carried out in several of the steps so as to sequence the oxidation and reduction phases in the following way:
- the redox potential is regulated so as to set up oxidizing conditions at one or more points of the process located upstream of the pasteurization phase;
- the redox potential is regulated so as to set up reducing conditions at one or more points of the process located downstream of the pasteurization.

4. Process according to Claim 2 or 3, **characterized in that** the fermented milk product is a yoghurt.

5. Process according to Claim 1, **characterized in that** said conducting of the process is done in such a way that, for at least one of said fermentation steps, controlled additions of a process gas make it possible to alternate between phases of the fermentation in question under reducing conditions and phases of the fermentation in question under oxidizing conditions.

6. Process according to Claim 5, **characterized in that** the process is a process for producing beer, and **in that** said conducting of the process allows the regulating of the redox potential during the fermentation to take place in two steps: in a first step, the fermentation takes place under regulated oxidizing conditions and in the presence of oxygen, so as to promote the growth of the yeast and a good physiological condition thereof, and in a second step, the redox potential is reduced to an optimal value so as to make it possible to improve the fermentation parameters, and also the sensory criteria.

7. Process according to Claim 5, **characterized in that** the process is a fermentation process in a fermenter, aimed at the production of biomass and/or of metabolites, and **in that** said conducting of the process makes it possible to regulate the redox potential of the medium at various successive values according to the various fermentation phases, so as to carry out a first phase under oxidizing conditions in order to promote the growth of the microbial strain through oxidizing conditions and, after a maximum biomass content has been obtained, to switch the fermenter to reducing conditions in order to initiate or intensify the production of one or more desired metabolites.

8. Process according to Claim 5, **characterized in that** the process is a fermentation process in a fermenter, aimed at the production of biomass and/or of metabolites, and **in that** said conducting of the process makes it possible to regulate the redox potential of the medium at various successive values according to the various fermentation phases, so as to carry out a first phase rendered slightly reducing, which is favorable to the growth of certain microorganisms and therefore to the production of a large biomass, followed by a more reducing phase which will make it possible to promote the production of desired flavor compounds.

9. Process according to Claim 5, **characterized in that** the process is a fermentation process in a fermenter, aimed at the production of biomass and/or of metabolites, and **in that** said conducting of the process makes it possible to modify and regulate the redox potential of the medium at a different value at the end of fermentation in order to adapt the metabolism or the physiology of the microorganisms so as to prepare them for a subsequent step.

10. Process according to Claim 5, **characterized in that** the process is a fermentation process in a fermenter, aimed at the production of biomass and/or of metabolites, and **in that** said conducting of the process makes it possible to change the redox potential level after the production of a desired metabolite so as to promote the excretion of the metabolite in question into the recovery medium.

11. Process according to Claim 5, **characterized in that** the process is a fermentation process in a fermenter, aimed at the production of biomass and/or of metabolites, and **in that** said conducting of the process makes it possible to change the redox potential level after the production of a precursor of a molecule of interest so as to promote a chemical reaction for obtaining the desired molecule of interest.

12. Process according to one of Claims 5 to 11,
**characterized in that** the process is a fermentation process in a fermenter, aimed at the production of biomass and/or of metabolites, and **in that**, subsequent to the fermentation, the following are carried out:
- centrifugation and/or filtration and/or ultrafiltration steps aimed at recovering the biomass produced, or
- purification steps on the fermentation medium in order to separate and concentrate metabolites,
and **in that** said conducting of the process makes it possible to change the redox potential level after the separation of the medium containing the molecule of interest from the biomass so as to promote the selective separation of the molecule of interest from the other compounds of the medium by promoting, for example, the binding to a resin either in the reduced form or in the oxidized form of the molecule of interest and elution thereof with a suitable solution.

## Patentansprüche

1. Verfahren zur Herstellung eines Nahrungsmittel- oder biotechnologischen Produkts unter Durchführung eines oder mehrerer Schritte, wobei einer oder mehrere der Schritte ein Medium einsetzen, wobei einer oder mehrere der Schritte, der/die ein Medium einsetzen, ein Fermentationsschritt ist, in dessen Verlauf man für mindestens den einen der Schritte des Verfahrens einen Arbeitsschritt zur Kontrolle des Redoxpotenzials des Mediums des betreffenden Schritts durchführt, und
**dadurch gekennzeichnet, dass** man die Steuerung des Verfahrens folgendermaßen durchführt:
- man reguliert das Redoxpotenzial des Mediums von dem Schritt, dessen Redoxpotenzial kontrolliert wird, mithilfe kontrollierter Zugaben eines Behandlungsgases in das betreffende Medium auf eine zuvor festgelegte Sollhöhe ein und
- man lässt das Übergehen des betreffenden Schritts zum folgenden Schritt in dem Verfahren zu, wenn der Sollwert erreicht worden ist,
so dass mindestens einer der Schritte des Verfahrens unter reduzierenden Bedingungen und mindestens einer der Schritte des Verfahrens unter oxidierenden Bedingungen durchgeführt wird.

2. Verfahren nach Anspruch 1, **dadurch gekennzeichnet, dass** es sich bei dem Verfahren um ein Verfahren zur Herstellung eines fermentierten Milchprodukts handelt, und **dadurch**, dass die Regulation des Redoxpotenzials in mehreren der Schritte derart stattfindet, dass die Oxidations- und Reduktionsphasen folgendermaßen aneinandergereiht werden:
- man führt eine Regulation des Redoxpotenzials derart durch, dass reduzierende Bedingungen an einem oder mehreren Punkten des Verfahrens eingesetzt werden, die stromaufwärts der Pasteurisierungsphase liegen;
- man führt eine Regulation des Redoxpotenzials derart durch, dass oxidierende Bedingungen an einem oder mehreren Punkten des Verfahrens eingesetzt werden, die stromabwärts der Pasteurisierungsphase liegen.

3. Verfahren nach Anspruch 1, **dadurch gekennzeichnet, dass** es sich bei dem Verfahren um ein Verfahren zur Herstellung eines fermentierten Milchprodukts handelt, und **dadurch**, dass die Regulation des Redoxpotenzials in mehreren der Schritte derart stattfindet, dass die Oxidations- und Reduktionsphasen folgendermaßen aneinandergereiht werden:
- man führt eine Regulation des Redoxpotenzials derart durch, dass oxidierende Bedingungen an einem oder mehreren Punkten des Verfahrens eingesetzt werden, die stromaufwärts der Pasteurisierungsphase liegen;
- man führt eine Regulation des Redoxpotenzials derart durch, dass reduzierende Bedingungen an einem oder mehreren Punkten des Verfahrens eingesetzt werden, die stromabwärts der Pasteurisierungsphase liegen.

4. Verfahren nach Anspruch 2 oder 3, **dadurch gekennzeichnet, dass** es sich bei dem fermentierten Milchprodukt um einen Joghurt handelt.

5. Verfahren nach Anspruch 1, **dadurch gekennzeichnet, dass** die Steuerung des Verfahrens derart durchgeführt wird, dass es bei wenigstens einem der Fermentationsschritte kontrollierte Zugaben eines Behandlungsgases ermöglichen, Phasen der betreffenden Fermentation unter reduzierenden Bedingungen mit Phasen der betreffenden Fermentation unter oxidierenden Bedingungen abzuwechseln.

6. Verfahren nach Anspruch 5, **dadurch gekennzeichnet, dass** es sich bei dem Verfahren um ein Verfahren zur Herstellung von Bier handelt, und **dadurch**, dass es die Steuerung des Verfahrens gestattet, dass die Regulation des Redoxpotenzials während der Fermentation zu zwei Zeitpunkten stattfindet: zu einem ersten Zeitpunkt erfolgt die Fermentation unter regulierten oxidierenden Bedingungen und in Gegenwart von Sauerstoff, damit das Wachstum der Hefe und ihr guter physiologischer Zustand gefördert werden, und zu einem zweiten Zeitpunkt wird das Redoxpotenzial auf einen optimalen Wert für die Verbesserung der Fermentationsparameter sowie der sensorischen Kriterien gesenkt.

7. Verfahren nach Anspruch 5, **dadurch gekennzeichnet, dass** es sich bei dem Verfahren um ein Fermentationsverfahren im Fermenter handelt, das auf die Herstellung von Biomasse und/oder Metaboliten abzielt, und **dadurch**, dass es die Steuerung des Verfahrens gestattet, das Redoxpotenzial des Mediums auf unterschiedliche, aufeinander folgende Werte je nach verschiedenen Phasen der Fermentation zu regulieren, so dass eine erste Phase unter oxidierenden Bedingungen durchgeführt wird, um das Wachstum des Mikrobenstamms durch oxidierende Bedingungen zu fördern, und anschließend die Gewinnung eines maximalen Gehalts an Biomasse, indem man den Fermenter in reduzierende Bedingungen absinken lässt, um die Produktion von einem oder mehreren gewünschten Metaboliten einzuleiten oder zu verstärken.

8. Verfahren nach Anspruch 5, **dadurch gekennzeichnet, dass** es sich bei dem Verfahren um ein Fermentationsverfahren im Fermenter handelt, das auf die Herstellung von Biomasse und/oder Metaboliten abzielt, und **dadurch**, dass es die Steuerung des Verfahrens gestattet, das Redoxpotenzial des Mediums auf unterschiedliche, aufeinander folgende Werte je nach verschiedenen Phasen der Fermentation zu regulieren, so dass eine erste, leicht reduzierend gemachte Phase durchgeführt wird, die für das Wachstum bestimmter Mikroorganismen und somit für die Produktion einer hohen Biomasse vorteilhaft ist, gefolgt von einer stärker reduzierenden Phase, die es gestattet, die Produktion gewünschter Aromaverbindungen zu fördern.

9. Verfahren nach Anspruch 5, **dadurch gekennzeichnet, dass** es sich bei dem Verfahren um ein Fermentationsverfahren im Fermenter handelt, das auf die Herstellung von Biomasse und/oder Metaboliten abzielt, und **dadurch**, dass es die Steuerung des Verfahrens gestattet, das Redoxpotenzial des Mediums am Ende der Fermentation auf einen anderen Wert zu verändern und zu regulieren, um den Stoffwechsel oder die Physiologie der Mikroorganismen anzupassen, damit sie für einen abschließenden Schritt vorbereitet werden.

10. Verfahren nach Anspruch 5, **dadurch gekennzeichnet, dass** es sich bei dem Verfahren um ein Fermentationsverfahren im Fermenter handelt, das auf die Herstellung von Biomasse und/oder Metaboliten abzielt, und **dadurch**, dass es die Steuerung des Verfahrens gestattet, die Höhe des Redoxpotenzials nach der Produktion eines gewünschten Metaboliten zu verändern, so dass die Exkretion des betreffenden Metaboliten in das Gewinnungsmedium begünstigt wird.

11. Verfahren nach Anspruch 5, **dadurch gekennzeichnet, dass** es sich bei dem Verfahren um ein Fermentationsverfahren im Fermenter handelt, das auf die Herstellung von Biomasse und/oder Metaboliten abzielt, und **dadurch**, dass es die Steuerung des Verfahrens gestattet, die Höhe des Redoxpotenzials nach der Produktion einer Vorstufe eines Moleküls von Interesse zu verändern, um eine chemische Reaktion zu fördern, welche die Gewinnung des gesuchten Moleküls von Interesse gestattet.

12. Verfahren nach einem der Ansprüche 5 bis 11, **dadurch gekennzeichnet, dass** es sich bei dem Verfahren um ein Fermentationsverfahren im Fermenter handelt, das auf die Herstellung von Biomasse und/oder Metaboliten abzielt, und **dadurch**, dass man nach der Fermentation Folgendes durchführt:
- Zentrifugations- und/oder Filtrations- und/oder Ultrafiltrationsschritte, die auf die Gewinnung der produzierten Biomasse abzielen, oder
- Reinigungsschritte an dem Fermentationsmedium, um die Metaboliten abzutrennen und zu konzentrieren,
und **dadurch**, dass es die Steuerung des Verfahrens gestattet, die Höhe des Redoxpotenzials nach der Abtrennung des Mediums, welches das Molekül von Interesse enthält, von der Biomasse zu verändern, um die selektive Abtrennung des Moleküls von Interesse von anderen Verbindungen des Mediums zu begünstigen, indem zum Beispiel die Bindung des Moleküls von Interesse entweder in reduzierter oder in oxidierter Form an ein Harz und seine Elution durch eine geeignete Lösung begünstigt werden.
